# EUROPEAN PATENT APPLICATION

(11) **EP 2 064 951 A1**
(43) Date of publication of application: **03.06.2009**
(21) Application number: 09004137.7
(22) Date of filing: 06.10.2000
(51) Int. Cl.: A01N 43/50, A61K 31/415, C07D 233/54, C07D 233/56, C07D 233/58

(54) **Use of a substituted imidazole, derivatives or salts thereof for the preparation of a medicament for the treatment of a cell proliferative disorder**

(30) Priority: 08.10.1999 US 158322 P
(62) Divisional of application: 00968797.1
(71) Applicant: Taiji Biomedical, Inc., Menlo Park CA 94025 (US)
(72) Inventor: Newman, Michael James, San Diego, CA 92130 (US); Dixon, William Ross, La Jolla, CA 92037 (US)
(74) Representative: Goldbach, Klara

(57) **Abstract**

The present invention relates to the use of a compound having the formula I, a derivative or a salt thereof for the preparation of a medicament for the treatment of a cell proliferative disorder, wherein the cell proliferative disorder is further treated by a chemotherapeutic agent

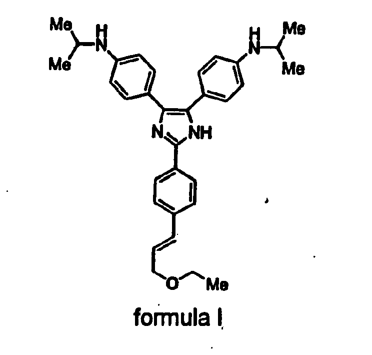

## Description

### FIELD OF THE INVENTION

The present invention relates generally to compositions and methods useful for treating cell proliferative disorders and more particularly to a method of enhancing the responsiveness of tumors to chemotherapy, increasing the dose of a chemotherapeutic agent that can be safely administered, and preventing the emergence of multidrug resistance (MDR). The present invention also provides a method for significantly enhancing oral bioavailability of chemotherapeutics.

### BACKGROUND

There are three major types of treatment currently in use to treat neoplasms: surgery, radiation therapy, and chemotherapy. Cytotoxic chemotherapeutic agents include a variety of natural products, for example taxanes, such as paclitaxel and docetaxel; vinca alkaloids such as vinblastine, vincristine and vinorelbine; anthracyclines such as doxorubicin, daunorubicin and idarubicin; and epipodophyllotoxins such as etoposide and teniposide. The ability of these agents to cure neoplastic disease is extremely limited due to lack of tumor cell specificity, the presence of multi-drug resistant (MDR) tumor cells at the time of first diagnosis and the *de novo* emergence of multi-drug resistant tumor cells during treatment.

Cytotoxic chemotherapeutic agents frequently suppress lymphocyte and hematopoietic and stem cell production, destroy the normal cells lining the digestive tract, and are toxic to the cardiovascular and nervous systems. These dose-limiting toxicities usually prevent the use of cytotoxic agents at doses which could kill sufficient numbers of tumor cells to effect a cure. The use of taxanes, vinca alkaloids, and anthracyclines is also largely limited to parenteral routes of administration, due to lack of oral bioavailability. This is due, in part, to the normal expression of a protein called P-glycoprotein (P-gp) in intestinal epithelial cells. P-gp is an ATP-dependent membrane transport protein that pumps certain absorbed xenobiotics, such as chemotherapeutic agents, back into the lumen of the digestive tract.

The most common mechanism of multidrug resistance (MDR) in tumor cells involves the aberrant over-expression of P-gp, resulting in transport of chemotherapeutic agents out of the tumor cells before they can kill the cell. P-gp can bind and transport taxanes, vinca alkaloids, anthracyclines and epipodophyllotoxins, to name a few, and its expression is sufficient to produce the MDR phenotype. P-gp expression has been found in many major tumor types at the time of first diagnosis, including acute myelogenous leukemia, breast, ovarian and colorectal carcinoma. In addition, P-gp expression in these tumor types increases after treatment of subjects with cytotoxic agents, via selection of pre-existing P-gp positive cells or spontaneous mutants expressing P-gp. Expression of P-gp in treated subjects contributes directly to therapeutic failure and relapse. For example, one study found that subjects with breast tumors expressing P-gp were three times more likely not to respond to chemotherapy than subjects whose tumors were P-gp negative. Drugs of proven antitumor chemotherapeutic value to which MDR has been observed include vinblastine, vincristine, etoposide, teniposide, doxorubicin (adriamycin), daunorubicin, taxanes, pliamycin (mithramycin), and actinomycin D (Jones *et al.,* Cancer (Suppl)1993, 72:3484-3488). Many tumors are intrinsically multidrug resistant (*e*.*g*., adenocarcinomas of the colon and kidney) while other tumors acquire MDR during the course of therapy (*e*.*g*., neuroblastomas and childhood leukemias).

Various agents have been described that inhibit P-gp and which may be used with cytotoxic chemotherapeutics in relapsed or refractory disease. Most of these agents exhibit intrinsic cytotoxicity and alter the pharmacokinetics of the co-administered cytotoxic agent, forcing a significant reduction in the amount of cytotoxic drug that can be administered. These properties are not readily compatible with use in newly diagnosed or therapy naïve subjects, even though this is the setting in which P-gp inhibition may be most effective.

### SUMMARY OF THE INVENTION

The present invention overcomes many of the problems discussed above by providing methods and compositions incorporating a compound having the general structure:

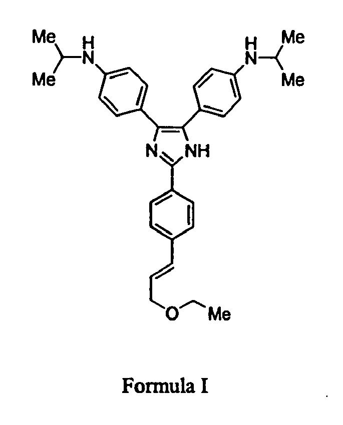

The compounds and methods described herein are capable of sensitizing tumor cells to antitumor chemotherapeutic agents, such as taxanes regardless of the expression of P-gp. They also have the ability to potentiate the sensitivity of tumor cells susceptible to these chemotherapeutic agents. The invention also provides a method of sensitizing naive or non-nave and/or multidrug-resistant tumor cells to antitumor chemotherapeutic agents. It also relates to a method of increasing the sensitivity of drug-susceptible tumor cells to antitumor chemotherapeutic agents. In addition, this invention relates to a method of inhibiting the emergence of MDR tumor cells during a course of treatment with antitumor chemotherapeutic agents. Finally, this invention relates to a method of reducing the effective dosage of an antitumor chemotherapeutic agent during a course of treatment.

In one embodiment, the invention provides a method of increasing the amount of a chemotherapeutic agent which can be safely administered to a subject. The method includes administering to a subject a compound having Formula I, derivatives or salts thereof and a chemotherapeutic agent at a dose equal to or above standard levels.

In another embodiment, the invention provides a method of treating a subject having a cell proliferative disorder including administering to a subject an effective amount of a chemotherapeutic agent and an effective amount of a compound having Formula I, derivatives or salts thereof thereby treating the subject.

In yet another embodiment, the invention provides a method of inhibiting the emergence of multi-drug resistant (MDR) neoplastic cells, including administering to a subject an effective amount of a chemotherapeutic agent and an effective amount of a compound having Formula I, derivatives or salts thereof, wherein the neoplastic cells do not express P-gp, do not express P-gp in all cells, or do not express P-gp at levels sufficient to manifest complete multi-drug resistance.

In another embodiment, the invention provides a method of protecting a subject from toxicity elicited by a chemotherapeutic agent, comprising administering to the subject a protective effective dose of a compound having Formula I, derivatives or salts thereof, thereby protecting the subject from the chemotherapeutic agent.

In yet another embodiment, the invention provides a method of increasing the amount of an anti-tumor chemotherapeutic agent which can be safely administered for treatment of a tumor. The method includes administering to a subject in need of such treatment a compound having Formula I, derivatives or salts thereof and a chemotherapeutic agent at a dose above standard levels.

### BRIEF DESCRIPTION OF THE FIGURES

**FIG. 1** is a graph showing the effect of the compound of Formula I on MDA/LCC6 and P-gp expressing MDA/LCC6^{MDRI} human breast carcinoma in response to paclitaxel *in vitro.*
**FIG. 2** is a graph showing the effect of the compound of Formula I on non-P-gp-expressing MDA/LCC6 human breast carcinoma xenografts in SCID mice *in vivo.*
**FIG. 3** is a graph showing the effect of the compound of Formula I on the toxicity and effect of paclitaxel in SCID mice *in vivo.*
**FIG. 4** is a graph showing the oral bioavailability and half-life of the compound of Formula I in canine sp.
**FIG. 5** is a linear graphical representations of the concentration of paclitaxel in plasma vs. time for the combination paclitaxel and Formula I compound mesylate salt.
**FIG. 6** is a graph demonstrating that pretreatment of mice with three oral doses of 30 mg/kg of the Formula I compound had no effect on i.v. plasma paclitaxel levels.

### DETAILED DESCRIPTION OF THE INVENTION

Cytotoxic agents are commonly used as antitumor chemotherapeutic agents. These agents are also called antiproliferative agents and chemotherapeutic agents. The desired effect of cytotoxic drugs is selective cell death with destruction of the malignant neoplastic cells while sparing normal cells.

Cytotoxic agents have also proved valuable in the treatment of other neoplastic disorders including connective or autoimmune diseases, metabolic disorders, dermatological diseases, and DNA virus infections.

Proper use of cytotoxic agents requires a thorough familiarity with the natural history and pathophysiology of the disease before selecting the cytotoxic agent, determining a dose, and undertaking therapy. Each subject must be carefully evaluated, with attention directed toward factors which may potentiate toxicity, such as overt or occult infections, bleeding dyscrasias, poor nutritional status, and severe metabolic disturbances. In addition, the functional condition of certain major organs, such as liver, kidneys, and bone marrow, is extremely important. Therefore, the selection of the appropriate cytotoxic agent and devising an effective therapeutic regimen is influenced by the presentation of the subject. Such considerations affect the dosage and type of drug administered.

Cytotoxic drugs as antitumor chemotherapeutic agents can be subdivided into several broad categories, including, (1) alkylating agents, such as mechlorethamine, cyclophosphamide, melphalan, uracil mustard, chlorambucil, busulfan, carmustine, lomustine, semustine, streptozoticin, and decrabazine; (2) antimetabolites, such as methotrexate, fluorouracil, fluorodeoxyuridine, cytarabine, azarabine, idoxuridine, mercaptopurine, azathioprine, thioguanine, and adenine arabinoside; (3) natural product derivatives, such as vinblastine, vincristine, dactinomycin, daunorubicin, doxorubicin, mithramycin, taxanes (e.g., paclitaxel) bleomycin, etoposide, teniposide, and mitomycin C; and (4) miscellaneous agents, such as hydroxyurea, procarbezine, mititane, and cisplatinum.

Important antitumor chemotherapeutic agents (with the usual effective dosage) to which clinical multidrug-resistance has been observed include vinblastine (0.1 mg per kilogram per week), vincristine (0.01 mg per kilogram per week), etoposide (35 to 50 mg per square meter per day), dactinomycin (0.15 mg per kilogram per day), doxorubicin (500 to 600 mg per square meter per week), daunorubicin (65 to 75 mg per square meter per week), and mithramycin (0.025 mg per kilogram per day). MDR has been shown to occur *in vitro* as well as in the clinic. Accordingly, by increasing the dosage of these drugs, one can increase the cytotoxic effect upon the tumor cells. The present invention provides methods whereby cytotoxic drug dosage can be increased without increasing the toxicity to the subject. The methods of the invention include administering substantial imidazoles, derivatives, analogues, and salts thereof in conjunction with a cytotoxic agent.

The substituted imidazoles, derivatives, analogs, and salts thereof having the general Formula: 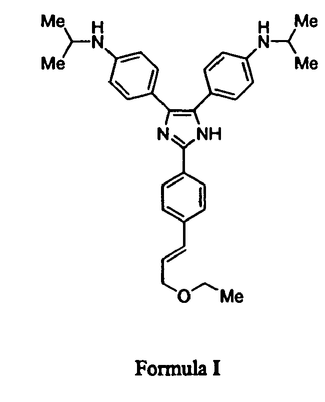 have been shown to inhibit P-gp function and are effective in reversing multidrug resistance in cells that express P-gp (see U.S. Patent Nos. 5,700,826 and 5,840,721, which are incorporated herein by reference in their entirety).

The present invention is based upon the discovery that the compound of Formula I, derivatives, analogues, and salts thereof, not only inhibit MDR in tumor cells expressing P-gp as described in U.S. Patents 5,700,826 and 5,840,721, but also allow the safe administration of selected chemotherapeutic agents at standard or even higher doses thought to be more toxic, to treat subjects, particularly subjects that are naïve to chemotherapeutic treatment. *In vivo* administration of the compound in combination with chemotherapeutic agents also enhances the therapeutic effect of cytotoxic agents against tumors that do not express P-gp, thus preventing the subsequent emergence of multidrug resistance.

The compound of Formula I, derivatives, and salts thereof, can be used to significantly enhance the bioavailability of orally administered carmustine (BCNU), chlorambucil (Leukeran), cisplatin (Platinol), Cytarabine, doxorubicin (Adriamycin), fluorouracil (5-FU), methoxetrate (Mexate), CPT111, etoposide, plicamycin (Mithracin) and taxanes such as, for example, paclitaxel.

The methods and compositions of the invention are useful for increasing the sensitivity of cells having cell proliferative disorders (*e*.*g*., a neoplasm) to chemotherapeutic agents such as, for example, paclitaxel. By increasing the efficacy without concomittant toxicity to non-cancer cells the invention provides methods and compositions useful for treating tumors and preventing or reducing the chances of relapse and death as a result of cytotoxicity. In addition, the invention eliminates or reduces the number of MDR cells by eliminating cancer cells prior to any mutation inducing an MDR phenotype or overproduction of P-gp conferring an MDR phenotype. Accordingly, by reducing multi-drug resistant tumor cells from arising, the invention satisfies the shortcomings of current therapeutic modalities.

The compound of Formula I has been found to be non-cytotoxic against normal or tumor cell lines at doses up to 100 µM and did not enhance the cytotoxic effect of chemotherapeutics against cells which do not express P-gp, under *in vitro* conditions. In addition, when the compound of Formula I was tested *in vivo* with co-administration of natural product chemotherapeutic agents such as, for example, paclitaxel, the compound of Formula I had no intrinsic anti-tumor activity and did not enhance the toxicity of co-administered paclitaxel. However, the compound of Formula I enhanced the anti-tumor effect of cytotoxic drugs such as paclitaxel against human tumor xenografts that did not express P-gp. This unexpected synergy was not the result of an effect of the compound of Formula I on paclitaxel blood levels.

In another embodiment, the invention provides a method of reducing the cytotoxic effects of chemotherapeutic agents on non-cancer cells. Co-administration of the compound of Formula I, derivatives, analogues, or salts thereof, with a chemotherapeutic agent (*e*.*g*., paclitaxel) reduced the toxicity of the chemotherapeutic agent to the subject. The compound of Formula I protected subjects from the lethal effects of high-dose chemotherapeutic therapy, such as paclitaxel therapy. Under these conditions, the compound of Formula I allows the administration of high enough doses of chemotherapeutic agents to completely inhibit the growth of tumors that do not express P-gp. Complete (100%) suppression of tumor growth was not observed with paclitaxel alone under any circumstances. The term "subject" as used herein refers to any animal having a cell proliferative disorder (*e*.*g*., a neoplastic disorder). Subjects for the purposes of the invention include, but are not limited to, mammals (*e*.*g*., bovine, canine, equine, feline, porcine) and preferably humans.

In one embodiment, the present invention provides a method for treating a subject having a cell proliferative disorder. The method includes administering a compound of Formula I, an analogue, derivative, or salt thereof, prior to, simultaneously with, or subsequent to administration of a chemotherapeutic agent. Preferably the subject is a naïve subject. A "naïve subject" is a subject that has not been treated with the same chemotherapeutic agent, and more preferably has not been treated with any chemotherapeutic agent. As described more fully below the compound of Formula I, an analogue, derivative, or its pharmaceutically acceptable salt (*e*.*g*., the mesylate salt) is administered. Preferably, the mesylate salt is administered.

By "cell proliferative disorder" is meant that a cell or cells demonstrate abnormal growth, typically aberrant growth, leading to a neoplasm, tumor or a cancer. Cell proliferative disorders include, for example, cancers of the breast, lung, prostate, kidney, skin, neural, ovary, uterus, liver, pancreas, epithelial, gastric, intestinal, exocrine, endocrine, lymphatic, hematopoietic system or head and neck tissue. Preferably the cancer does not necessarily express P-gp. More generally, neoplastic diseases are conditions in which abnormal proliferation of cells results in a mass of tissue called a neoplasm or tumor. Neoplasms have varying degrees of abnormalities in structure and behavior. Some neoplasms are benign while others are malignant or cancerous. An effective treatment of neoplastic disease would be considered a valuable contribution to the search for cancer preventive or curative procedures.

For example, in one embodiment a compound of Formula I, an analogue, derivative, or its pharmaceutically acceptable salt, *e*.*g*., the mesylate salt, is administered to a subject who has not previously been exposed to chemotherapy and who has cells having a cell proliferative disorder which do not express P-gp. Under these conditions, administration of the compound of Formula I, analogue derivative, or salt thereof, enhances the anti-tumor activity of a co-administered cytotoxic agent, such as a taxane. The dose and efficacy of the compound of Formula I and the chemotherapeutic agent would be in an effective amount to inhibit MDR in any subpopulation of tumor cells expressing P-gp, and/or prevent the emergence of P-gp expressing cells. The methods of the invention allow the safe use of doses of chemotherapeutic agents which are, by themselves, often unacceptably toxic, the combination of which produce an increased remission rate. As used herein, an "effective amount" is that amount capable of inhibiting or modulating cell growth activity of neoplastic cells.

The compound of Formula I enhances the activity of drugs belonging to, for example, the taxane, vinca alkaloid, anthracycline, and (or) epipodophyllotoxin classes of anti-tumor agents, in addition to dactinomycin, mithromycin, and (or) mitomycin, all such agents being administered by standard routes and regimens.

In another embodiment, the invention provides compositions and methods useful to protect a subject from the cytotoxic effects of chemotherapeutic agents. The method includes administering to a subject a protective effective amount of a compound having Formula I, analogues, derivatives or salts thereof. The compound of Formula I reduces the lethality of the chemotherapeutic agent on the organism. The compound of Formula I protected subjects from the lethal effects of high-dose chemotherapeutic therapy, such as, for example, paclitaxel therapy. Under these conditions, the compound of Formula I allows the administration of high enough doses of chemotherapeutic agents to completely inhibit the growth of tumors that do not express P-gp.

Under conditions where the compound of Formula I analogue, derivative, or salt thereof is administered to protect a subject from chemotherapeutic agent toxicity, the method of treatment includes standard doses as well as an increase above each standard dose by about 25 to 100%, more preferably, about 50 to 100%. Thus, for example, paclitaxel is given as a 1-hour, 3-hour, or 24-hour intravenous infusion at doses from 80 to 225 mg/m² either once per week or every three weeks. In conjunction with the compound of Formula I, the paclitaxel dose could be increased from approximately 1.25 to 3-fold, depending on the regimen.

Accordingly, the invention provides methods and compositions useful for the prevention or the emergence of multi-drug resistant tumors during the course of cancer treatment. These same compounds additionally provide a method for reducing the required dosage of an antitumor chemotherapeutic agent.

The methods of this invention involve in one embodiment, (1) the administration of a compound of Formula I, an analogue, derivative, or salt thereof, prior to, together with, or subsequent to the administration of an antitumor chemotherapeutic agent; or (2) the administration of a combination of a compound of Formula I and an antitumor chemotherapeutic agent.

Thus, the compound of Formula I analogues, derivatives, or salts thereof are useful in the treatment of multidrug-resistant tumor cells or tumor cells in general, either separately or in combination with an antitumor chemotherapeutic agent. These compounds may be administered orally, topically or parenterally in dosage unit formulations containing conventional non-toxic pharmaceutically acceptable carriers, adjuvants, and vehicles. The term parenteral as used herein includes subcutaneous injections, aerosol, intravenous, intramuscular, intrathecal, intracranial, intrasternal injection or infusion techniques.

The present invention also has the objective of providing suitable topical, oral, and parenteral pharmaceutical formulations for use in the novel methods of treatment of the present invention. The compounds of the present invention may be administered orally as tablets, aqueous or oily suspensions, lozenges, troches, powders, granules, emulsions, capsules, syrups or elixirs. The composition for oral use may contain one or more agents selected from the group of sweetening agents, flavouring agents, colouring agents and preserving agents in order to produce pharmaceutically elegant and palatable preparations. The tablets contain the acting ingredient in admixture with non-toxic pharmaceutically acceptable excipients which are suitable for the manufacture of tablets. These excipients may be, for example, (1) inert diluents, such as calcium carbonate, lactose, calcium phosphate or sodium phosphate; (2) granulating and disintegrating agents, such as corn starch or alginic acid; (3) binding agents, such as starch, gelatin or acacia; and (4) lubricating agents, such as magnesium stearate, stearic acid or talc. These tablets may be uncoated or coated by known techniques to delay disintegration and absorption in the gastrointestinal tract and thereby provide a sustained action over a longer period. For example, a time delay material such as glyceryl monostearate or glyceryl distearate may be employed. Coating may also be performed using techniques described in the U.S. Pat. Nos. 4,256,108; 4,160,452; and 4,265,874 to form osmotic therapeutic tablets for control release.

The compound of Formula I, analogues, derivatives, or salts thereof, as well as the chemotherapeutic agents useful in the method of the invention can be administered, for *in vivo* application, parenterally by injection or by gradual perfusion over time independently or together. Administration may be intravenously, intraperitoneally, intramuscularly, subcutaneously, intracavity, or transdermally. For *in vitro* studies the agents may be added or dissolved in an appropriate biologically acceptable buffer and added to a cell or tissue.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, growth factors and inert gases and the like.

It is envisioned that the invention can be used to treat pathologies associated cell proliferative disorders, including, for example, neoplasms, cancers (*e*.*g*., cancers of the breast, lung, prostate, kidney, skin, neural, ovary, uterus, liver, pancreas, epithelial, gastric, intestinal, exocrine, endocrine, lymphatic, hematopoietic system or head and neck tissue), fibrotic disorders and the like. Therefore, the present invention encompasses methods for ameliorating a disorder associated with cell proliferation, neoplasms, cancers and the like, including treating a subject having the disorder, at the site of the disorder, with a compound of Formula I, analogues, derivatives, or salts thereof and a chemotherapeutic agent in an amount sufficient to inhibit or ameliorate the cell's proliferation or the disorder. Generally, the terms "treating", "treatment" and the like are used herein to mean affecting a subject, tissue or cell to obtain a desired pharmacologic and/or physiologic effect. The effect may be prophylactic in terms of completely or partially preventing a cell proliferative disorder or sign or symptom thereof, and/or may be therapeutic in terms of a partial or complete cure for a disorder and/or adverse effect attributable to, for example, aberrant cell proliferation. "Treating" as used herein covers any treatment of, or prevention of a cell proliferative disorder in a vertebrate, a mammal, particularly a human, and includes: (a) preventing the disorder from occurring in a subject that may be predisposed to the disorder, but has not yet been diagnosed as having it; (b) inhibiting the disorder, *i*.*e*., arresting its development; or (c) relieving or ameliorating the disorder, *i*.*e*., cause regression of the disorder.

The invention includes various pharmaceutical compositions useful for ameliorating cell proliferative disorder, including neoplams, cancers and the like. The pharmaceutical compositions according to one embodiment of the invention are prepared by bringing a compound of Formula I, analogue, derivatives or salts thereof and one or more chemotherapeutic agents or combinations of the compound of Formula I and one or more chemotherapeutic agents into a form suitable for administration to a subject using carriers, excipients and additives or auxiliaries. Frequently used carriers or auxiliaries include magnesium carbonate, titanium dioxide, lactose, mannitol and other sugars, talc, milk protein, gelatin, starch, vitamins, cellulose and its derivatives, animal and vegetable oils, polyethylene glycols and solvents, such as sterile water, alcohols, glycerol and polyhydric alcohols. Intravenous vehicles include fluid and nutrient replenishers. Preservatives include antimicrobial, anti-oxidants, chelating agents and inert gases. Other pharmaceutically acceptable carriers include aqueous solutions, non-toxic excipients, including salts, preservatives, buffers and the like, as described, for instance, in Remington's Pharmaceutical Sciences, 15th ed. Easton: Mack Publishing Co., 1405-1412, 1461-1487 (1975) and The National Formulary XIV., 14th ed. Washington: American Pharmaceutical Association (1975), the contents of which are hereby incorporated by reference. The pH and exact concentration of the various components of the pharmaceutical composition are adjusted according to routine skills in the art. See Goodman and Gilman's The Pharmacological Basis for Therapeutics (7th ed.).

The pharmaceutical compositions are preferably prepared and administered in dose units. Solid dose units are tablets, capsules and suppositories. For treatment of a subject, depending on activity of the compound, manner of administration, nature and severity of the disorder, age and body weight of the subject, different daily doses can be used. Under certain circumstances, however, higher or lower daily doses may be appropriate. The administration of the daily dose can be carried out both by single administration in the form of an individual dose unit or else several smaller dose units and also by multiple administration of subdivided doses at specific intervals.

The pharmaceutical compositions according to the invention may be administered locally or systemically in a therapeutically effective dose. Amounts effective for this use will, of course, depend on the severity of the disease and the weight and general state of the subject. Typically, dosages used *in vitro* may provide useful guidance in the amounts useful for *in situ* administration of the pharmaceutical composition, and animal models may be used to determine effective dosages for treatment of particular disorders. Various considerations are described, *e*.*g*., in Langer, Science, 249:1527, (1990); Gilman *et al.* (eds.) (1990), each of which is herein incorporated by reference.

The method by which the compound of Formula I may be administered for oral use would be, for example, in a hard gelatin capsule wherein the active ingredient is mixed with an inert solid diluant, or soft gelatin capsule, wherein the active ingredient is mixed with a cosolvent mixture, such as PEG 400 containing Tween-20. A compound of Formula I may also be administered in the form of a sterile injectable aqueous or oleaginous solution or suspension. The active ingredient can be administered intravenously or as an oral dose of 0.5 to 10 mg/kg given every 12 hours, 1 to 3 times before and 1 to 3 times after the administration of the chemotherapeutic agent, with at least one dose 1 to 4 hours before and at least one dose within 8 to 12 hours after the administration of the chemotherapeutic agent.

Formulations for oral use may be in the form of hard gelatin capsules wherein the active ingredient is mixed with an inert solid diluent, for example, calcium carbonate, calcium phosphate or kaolin. They may also be in the form of soft gelatin capsules wherein the active ingredient is mixed with water or an oil medium, such as peanut oil, liquid paraffin or olive oil.

Aqueous suspensions normally contain the active materials in admixture with excipients suitable for the manufacture of aqueous suspension. Such excipients may be (1) suspending agent such as sodium carboxymethyl cellulose, methyl cellulose, hydroxypropylmethylcellulose, sodium alginate, polyvinylpyrrolidone, gum tragacanth and gum acacia; (2) dispersing or wetting agents which may be (a) naturally occurring phosphatide such as lecithin; (b) a condensation product of an alkylene oxide with a fatty acid, for example, polyoxyethylene stearate; (c) a condensation product of ethylene oxide with a long chain aliphatic alcohol, for example, heptadecaethylenoxycetanol; (d) a condensation product of ethylene oxide with a partial ester derived from a fatty acid and hexitol such as polyoxyethylene sorbitol monooleate, or (e) a condensation product of ethylene oxide with a partial ester derived from fatty acids and hexitol anhydrides, for example polyoxyethylene sorbitan monooleate.

The pharmaceutical compositions may be in the form of a sterile injectable aqueous or oleagenous suspension. This suspension may be formulated according to known methods using those suitable dispersing or wetting agents and suspending agents which have been mentioned above. The sterile injectable preparation may also a sterile injectable solution or suspension in a non-toxic parenterally-acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose, any bland fixed oil may be employed including synthetic mono- or diglycerides. In addition, fatty acids such as oleic acid find use in the preparation of injectables.

A compound of Formula I may also be administered in the form of suppositories for rectal administration of the drug. These compositions can be prepared by mixing the drug with a suitable non-irritating excipient which is solid at ordinary temperature but liquid at the rectal temperature and will therefore melt in the rectum to release the drug. Such materials are cocoa butter and polyethylene glycols.

The compounds of the present invention may also be administered in the form of liposome delivery systems, such as small unilamellar vesicles, large unilamellar vesicles, and multilamellar vesicles. Liposomes can be formed from a variety of phospholipids, such as cholesterol, stearylamine, or phosphatidylcholines.

For topical use, creams, ointments, jellies, solutions or suspensions, etc., containing the compounds of Formula I are employed.

Dosage levels of the compounds of the present invention are of the order of about 0.5 mg to about 10 mg per kilogram body weight, with a preferred dosage range between about 5 mg to about 20 mg per kilogram body weight per day (from about 0.3 gms to about 1.2 gms per patient per day). The amount of active ingredient that may be combined with the carrier materials to produce a single dosage will vary depending upon the host treated and the particular mode of administration. For example, a formulation intended for oral administration to humans may contain about 5 mg to 1 g of an active compound with an appropriate and convenient amount of carrier material which may vary from about 5 to 95 percent of the total composition. Dosage unit forms will generally contain between from about 5 mg to 500 mg of active ingredient.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

In addition, some of the compounds of the instant invention may form solvates with water or common organic solvents. Such solvates are encompassed within the scope of the invention.

In yet another embodiment, the present invention provides compositions and methods whereby chemotherapeutic agents such as, for example, taxanes (e.g., paclitaxel), vinca alkaloids, anthracyclines, and epidophyllotoxins could be administered orally in a therapeutically effective manner. The studies provided herein (*e*.*g*., see Examples) indicate that co-administration of a Formula I compound with a chemotherapeutic agent, such as paclitaxel, enhances the oral bioavailability of such agents. Oral co-administration of agents such as, for example, paclitaxel, in an appropriate vehicle, with a compound of Formula I results in therapeutically useful blood levels of the chemotherapeutic agent. The method by which the cytotoxic chemotherapeutic agent may be administered for oral use would be, for example, in solution as a microemulsion, in a hard gelatin capsule wherein the active ingredient is mixed with an inert solid diluant, in a soft gelatin capsule wherein the active ingredient is dissolved in a cosolvent mixture such as PEG 400 containing Tween-20 and ethanol, or as a solid dispersion contained in a hard gelatin capsule. Thus, for example, paclitaxel can be given orally at a dose of 2 to 20 mg/kg once every week or once every three weeks, with co-administration of the compound of Formula I at an oral dose of 2 to 10 mg/kg, formulated in a hard gelatin capsule wherein the active ingredient is mixed with an inert solid diluant, or in a soft gelatin capsule, wherein the active ingredient is dissolved in a cosolvent mixture, such as PEG 400 containing Tween-20.

In yet another embodiment, the invention provides a method of treatment or preventing MDR or drug-resistant tumor cells by administering a sufficient amount of a compound of Formula I, derivatives and salts thereof, prior to, together with, or subsequent to the administration of an antitumor chemotherapeutic agent. As described more fully below, administration of the compound of Formula I and a chemotherapeutic agent results in the suppression of tumor growth by at least 50%; preferably 60%; and, more preferably, greater than 70%. Accordingly, the elimination of tumor growth and proliferation eliminates the production of MDR tumor cells reducing the recurrence of cancer and increasing the efficacy of chemotherapeutic treatments. A further object is to provide pharmaceutical compositions for increasing the sensitivity of tumor cells to antitumor chemotherapeutic agents and thus for the treatment of tumors that are susceptible to anti-cancer chemotherapeutic agents but have become resistant to such chemotherapy. Thus, the invention provides compositions and methods, which not only inhibits MDR in tumor cells expressing P-gp, but also allows the safe administration of chemotherapeutic agents at standard or even higher doses to treat naïve subjects, enhancing the therapeutic effect of chemotherapeutic agents against tumors that do not express P-gp and simultaneously preventing the subsequent emergence of multidrug resistance.

In addition, the invention provides methods whereby the compound of Formula I can be used to significantly enhance the bioavailability of orally administered chemotherapeutic agents including, for example, taxanes, such as paclitaxel.

The compound of Formula I, includes a family of novel substituted imidazole derivatives that are effective in increasing the sensitivity of tumor cells resistant to anticancer chemotherapeutic agents, such as doxorubicin (DOX), taxanes (e.g., paclitaxel), vinblastine (VLB), and enhancing the sensitivity of multi-drug resistant cells. These compounds have the effect of reducing the resistance of MDR tumor cells, and potentiating the sensitivity of cells to antitumor drugs, such as, for example, DOX, taxol, VLB.

A compound useful in the methods of this invention has the general structure as depicted in Formula I:

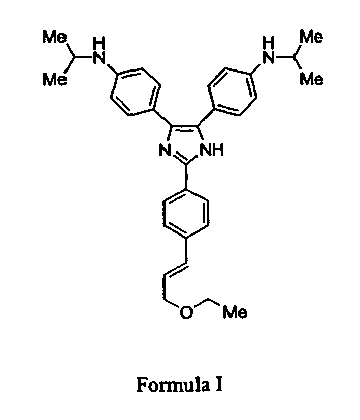

Derivatives and analogues of the compound of Formula I useful in the present invention include, for example, 2-[4-(3-ethoxy-trans-1-propen-1-yl)phenyl]-4,5-bis (4-N,N-diethylaminophenyl) imidazole; 2-[4-(3-ethoxy-trans-1-propen-1-yl)phenyl]-4-(4-N,N-diethylaminophenyl)-5-( 4-N-methylaminophenyl) imidazole; 2-[4-(3-methoxy-trans-1-propen-1-yl)phenyl]-4,5-bis (4-pyrrolidinophenyl) imidazole; 2-[4-(3-ethoxy-trans-1-propen-1-yl)phenyl]-4,5-bis (4-pyrrolidinophenyl) imidazole; 2-[4- (3-ethoxy-trans-1-propen-1-yl)phenyl]-4-(4-N-dimethylaminophenyl)-5-(4-pyrrolidinophenyl) imidazole; 2-[4-(3-ethoxy-trans-1-propen-1-yl)phenyl]-4-(4-N-methylaimnophenyl) -5-(4-pyrrolidinophenyl) imidazole; 2-[4-(3-ethoxy-trans-1-propen-1-yl)phenyl]-4,5-bis (4-N-morpholinophenyl) imidazole; 2-[4-(3-ethoxy-trans-1-propen-1-yl)phenyl]-4-(4-N-dimethylaminophenyl)-5- (4-N-morpholinophenyl) imidazole; 2-[4-(3-ethoxy-trans-1-propen-1-yl)phenyl]-4-(4-N-methylaminophenyl)-5-(4-N-morpholinophenyl) imidazole; and 2-[4-(3-ethoxy-trans-1-propen-1-yl)phenyl]-4-(4-N-methylaminophenyl)-5-(4-N - isopropylaminophenyl) imidazole.

Preferred compositions useful in the present invention include compositions comprising compounds as defined above in Formula I and derivatives thereof (or pharmaceutically acceptable salts, prodrugs, esters, or solvates of these compounds) in admixture with a pharmaceutically acceptable diluent, adjuvent, or carrier.

The compounds of Formula I, derivatives and salts thereof maybe prepared by procedures known to those skilled in the art from known compounds or readily preparable intermediates (see, for example, U.S. Patent No. 5,840,721, the disclosure of which is incorporated herein by reference).

The invention will now be described in greater detail by reference to the following non-limiting examples.

### EXAMPLES

Multidrug-resistant cell lines are easily obtainable for *in vitro* determination of drug sensitization and treatment by compounds of the present invention. *In vitro* potentiation of antineoplistic cytotoxicity by the imidazole derivatives of the present invention can be measured, for example, in both CEM/VLB 1000 and SK/VLB 1000 cell lines. These multidrug resistant cell lines can be obtained from Dr. Victor Ling, Ontario Cancer Institute, Toronto, Canada. The CEM/VLB 1000 cell line was maintained as a suspension in minimum essential medium supplemented with 10% fetal bovine serum in a humidified atmosphere of 95% air and 5% CO₂ while the SK/VLB 1000 cell line was maintained as adherent cells using the identical medium conditions as the CEM cells. The CEM/VLB 1000 cells are typically seeded at a density of 5 x 10⁴ cells/well in a 96 well microtiter plate while the SK/VLB 1000 cell line is typically seeded at a density of 2,500 cells/well after trypsinization. Vinblastine (5 µg/mL, for the CEM cells) or Taxol (3 µg/mL, for the SK cells) and the compound of Formula I (0.01 to 50 µM) can be added directly to the wells. After an incubation of 72 hours in presence of drug, alamar blue (B. Page et al., Int. J. OncoL 3:473-476, 1993) is added (10 µL to the 200 µL cell suspension) for a period of 4-6 hours after which the fluorescence (excitation=530 nM, emission=590 nM) is read for each well using a "CytoFluor" microtiter fluorometer plate reader. This assay measures the effective concentration of compound necessary to enhance the cytotoxicity (EC₅₀) of vinblastine or taxol in the MDR cell line.

***In Vivo* Antitumor Efficacy Models.** Anti-tumor efficacy experiments with orthotopic MDA/LCC6 and MDA/LCC6^{MDR1} tumors were conducted in female SCID/RAG2 mice. Ascites propagated cells (2 x 10⁶ in 50 µl) were injected into mammary fat pads bilaterally on day 0 before randomization into groups of 5 mice per group. Paclitaxel (12 mg/kg/dose) was administered QD i.v. (tail vein) in 200 µl saline/Cremophor/ethanol (8/1/1 by volume) on days 5, 12, 19, and 26. The compound of Formula I-free base (30 mg/kg/dose) was administered BID p.o. by gavage in 100 µl PEG 400/Tween 20 (9/1 by volume) on days 4-6, 11-13, 18-20, and 25-27. Mean body weights were recorded at least every other day. Tumor weight was monitored approximately every other day by caliper measurements and calculated according to the formula (Tumor weight = (length x width²) ÷ 2). This conversion formula was verified by comparing the calculation derived tumor weights to excised and weighed tumors. Animals bearing ulcerated tumors or where tumor weight exceeded 10% of the animal's body weight were terminated. The weights of the bilateral tumors were averaged for each mouse and mean tumor weights for each treatment group ± standard error of the mean were calculated. Statistical analysis was carried out by Mann-Whitney test using GraphPad Prism software (San Diego, CA).

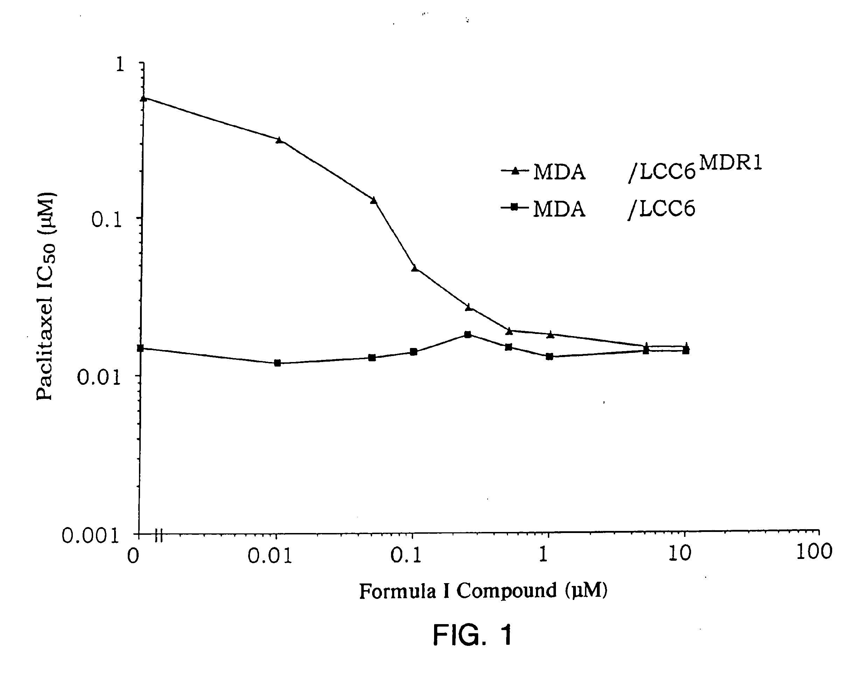

The compound of Formula I was administered the afternoon before, 2 hours before and 6 hours after each paclitaxel dose. The ability of orally administered Formula I compound to reverse MDR in solid tumors *in vivo* was assessed with the MDA/LCC6^{MDR1} model. Because the compound of Formula I produced no effect on paclitaxel blood levels, focus on up-front therapy or minimal residual disease models was performed, as opposed to established tumors. MDA/LCC6^{MDR1} cells that express P-gp were resistant to paclitaxel treatment *in vitro* and this resistance was reversed by the compound of Formula I (Figure 1). Treatment with paclitaxel or the compound of Formula I alone had no significant effect on *in vivo* tumor growth compared to the vehicle control (see, for example, Newman et al., Ca. Res. 60:2964, 2000, which is incorporated by herein by reference in its entirety). When the Formula I compound and paclitaxel were combined, there was a statistically significant inhibition of tumor growth that persisted for at least two weeks after the last dose of paclitaxel. The growth delay produced by paclitaxel and the Formula I compound in the MDA/LCC6^{MDR1} xenografts was comparable to the growth delay produced by paclitaxel alone in MDA/LCC6 xenografts. Similar results were obtained in three independent experiments with two different strains of immunocompromised mice (SCID/RAG-2 and athymic). Similar results were obtained with non-established and established tumors.

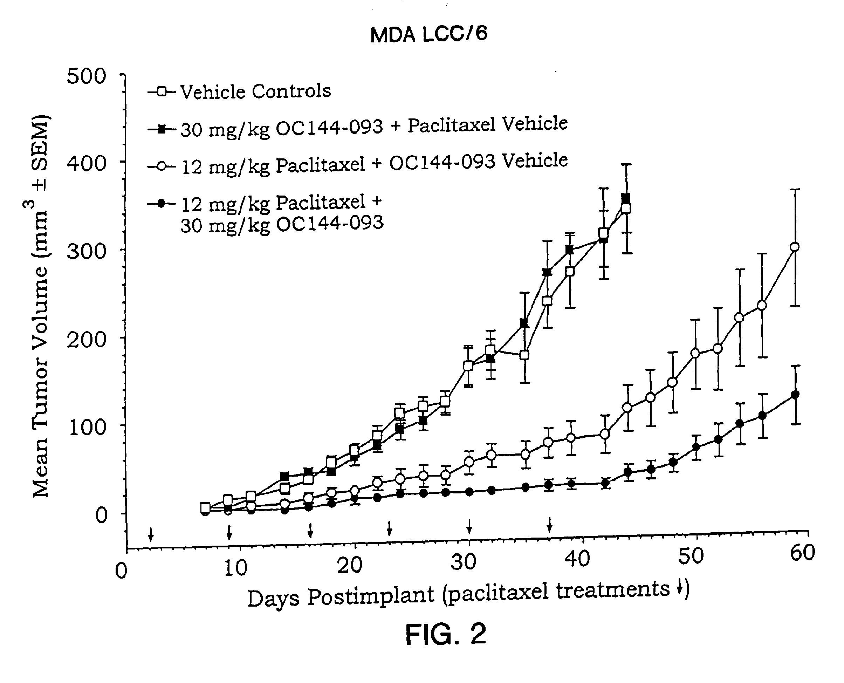

The compound of Formula I did not enhance the *in vitro* anti-tumor activity of paclitaxel against breast carcinoma cells that do not express P-gp (Figure 1). Surprisingly, the compound of Formula I did enhance the *in vivo* anti-tumor activity of paclitaxel against the same non-P-gp expressing tumor cells (Figure 2).

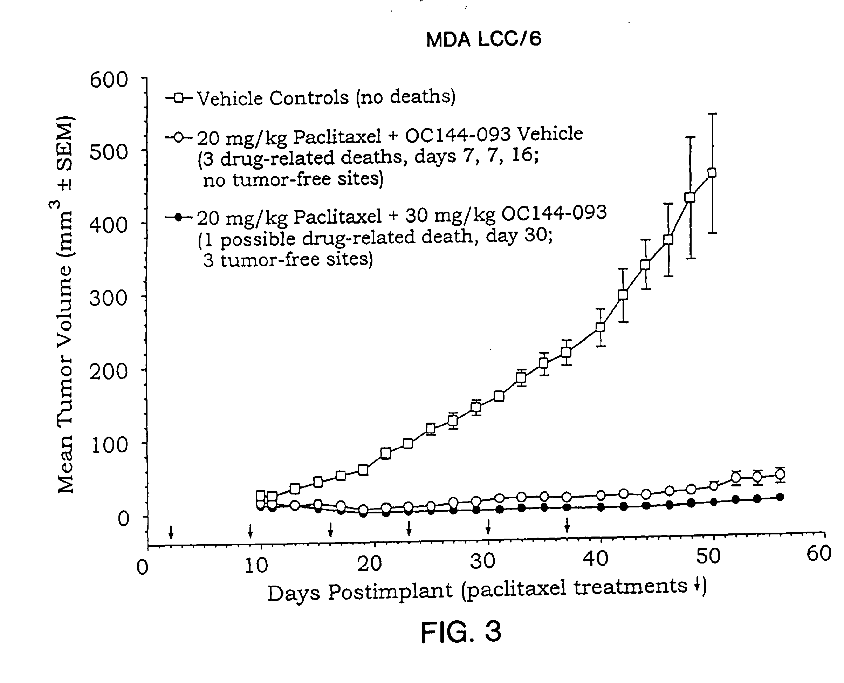

Non-tumor bearing athymic mice were treated with the compound of Formula I and (or) paclitaxel as described. The compound of Formula I decreased paclitaxel toxicity in the athymic mice independent of tumor presence (see Table I). The ability of the compound of Formula I to protect mice from lethal doses of paclitaxel allowed administration to tumor-bearing mice at doses higher than standard levels (Figure 3). Administration of 20 mg/kg paclitaxel alone once per week resulted in 50% lethality, with 3 of 6 animals dying within 16 days. This high LD₅₀ dose of paclitaxel significantly inhibited tumor growth, but did not completely prevent tumor formation at any injection site. Administration of 20 mg/kg paclitaxel with the compound of Formula I reduced lethality to one possible drug-related death and completely prevented tumor formation at several injection sites (Figure 3).

| Table I: *Effect of Paclitaxel Toxicity in Athymic Mice in the Presence and Absence of Formula I Compound* | |
|---|---|
| **Treatment** | **Survival** |
| Vehicle + Formula I compound | 4/4 |
| Paclitaxel + Vehicle | 0/4 |
| Paclitaxel + Formula I compound | 4/4 |
| *Paclitaxel: 24 mglkg i. v. Q5D x 6 +* 36 *mg*/*kg i.v. Q5D x2* | |
| *Compound of Formula I: 30 mg*/*kg p.o. afternoon before and BID day of paclitaxel treatments* | |

**Modulation of P-gp-Mediated Drug Resistance *In Vitro.*** the compound of Formula I was able to reverse resistance to all classes of P-gp substrates in a wide variety of tumor cell types with EC₅₀s in the low nM range (Table II). Similar results were obtained with etoposide in several models. Complete reversal of MDR was typically seen with Formula I doses between 0.25 and 1.0 µM. Examples with cells expressing extremely high levels of P-gp as a result of drug selection (CEM/VLB1000), moderate levels of P-gp from gene transduction (MDA/LCC6^{MDR1}), and low (intrinsic) levels of P-gp (HCT-15) are illustrated in Table II. The compound of Formula I had no effect on doxorubicin or paclitaxel IC₅₀s in non-P-gp-expressing CCRF-CEM and NMA/LCC6 cells, respectively (Figure 1, for MDA/LCC6 cells). The Formula I compound retained full MDR reversal potency after incubation in human plasma, suggesting that protein binding mediated-inactivation will not be a problem in humans.

| Table II *Reversal of MDR by the Compound of Formula I in P-gp-expressing Cell Lines* | | | |
|---|---|---|---|
| The IC₅₀s for the indicated anti-tumor agents were determined in the presence of various concentrations of the Formula I compound as described. The Formula I compound EC₅₀ is the concentration that produced half maximal reversal of anti-tumor agent resistance. Each experiment was carried out two to four times. | | | |
| Cell Line | Formula I compound EC₅₀ (µM ± SD) | | |
| | Doxorubicin | Vinblastine | Paclitaxel |
| CEM/VLB 1000^{a} | 0.09 ± 0.06 | 0.07 ± 0.01 | |
| MES-SA/DX5^{b} | 0.024 ± 0.006 | 0.034 ± 0.007 | 0.027 ± 0.007 |
| SK/VLB1000^{c} | 0.025 ± 0.008 | 0.015 | 0.033 |
| MCF-7/ADR^{d} | 0.038 ± 0.006 | 0.02 ± 0.01 | 0.031 ± 0.004 |
| MDA/LCC6^{MDR1d} | 0.013 | | 0.009 |
| HCT-15^{e} | | | .0016 |

| | | | |
|---|---|---|---|
| ^{a}lymphoma, ^{b}unterine, ^{c}ovarian, ^{d}breast, ^{e}colorectal carcinoma | | | |

**Non-Specific Toxicity of the Compound of Formula I/Cell Proliferation Assays.** Cell proliferation IC₅₀s and MDR reversal EC₅₀s were determined from 3-day dose-response curves carried out in triplicate in 96-well plates essentially as described by Monks et al. (J. Natl. Cancer Inst., 83:757-66, 1991). Cells were plated in standard growth medium at 2.5 or 5.0 x 10⁴ per well (CCRF-CEM and CEM/VLB1000 respectively) or 5 x 10³ per well (all other cell lines) in a final volume of 100 µl. After a 2 h incubation for non-adherent cells and overnight incubation for adherent cells, the initial cell density was determined by fluorescence readout of Alamar Blue metabolism. The compound of Formula I, cytotoxic agents, or compound vehicles were added to duplicate plates and the incubation was continued for an additional 72 h. Final cell density was determined with Alamar Blue. In some experiments, a standard endpoint assay was used without analysis of initial cell density with Alamar Blue. Similar results were obtained with the two assays. EC₅₀s were derived by nonlinear regression analysis assuming a sigmoidal dose-response using CrraphPad Prism Software (San Diego, CA). Proliferation assays capable of measuring both cytostatic and cytotoxic responses were carried out as described herein with 15 non-transformed and transformed cell lines, including primary fibroblasts, non-transformed smooth muscle, leukemia, breast, colon, ovarian and uterine carcinoma cells (± P-gp expression). Although the compound of Formula I reversed P-gp-mediated MDR in the low nanomolar concentration range, the compound was non-cytotoxic by itself at doses up to 100 µM in all cell lines. Cytostatic IC₅₀s ranged from 6 to 170 µM, with an average value of 60 µM (Table III). IC₅₀s for non-specific cytotoxicity were similar in matched cell lines plus and minus P-gp expression. The results indicate that the compound of Formula I is probably not a P-gp transport substrate. If it were, some consistent degree of resistance would be expected in P-gp expressing cells.

| Table III *Effect of the Compound of Formula I on the Proliferation of Various Cell Lines* | | | |
|---|---|---|---|
| The IC₅₀ for inhibition of the growth of cell lines by the compound of Formula I was determined as described. Each experiment was carried out at least two times with similar results. | | | |
| Human Cell Type/Line | | P-gp^{a} | |
| | | Expression | Formula I compound |
| | | | IC₅₀ |
| Primary fibroblast | | | |
| | CCD-986SK | | >100 |
| Smooth muscle | | | |
| | HISM | | >100 |
| Lymphoma | | | |
| | CEM | Neg | 38 |
| | CEM/VLB1000 | Pos | 32 |
| Ovarian carcinoma | | | |
| | SKOV3 | Neg | 48 |
| | SK/VLB1000 | Pos | 15 |
| Uterine carcinoma | | | |
| | MES-SA | Neg | 51 |
| Breast carcinoma | | | |
| | MCF-7 | Neg | 30 |
| | MCF-7/ADR | Pos | 68 |
| | MDA/LCC6 | Neg | >100 |
| | MDA/LCC6^{MDR1} | Pos | >100 |
| Colorectal carcinoma | | | |
| | SW480 | Pos | >100 |
| | SW620 | Neg | >100 |
| | HT-29 | Neg | 12 |
| | HCT-15 | Pos | 6 |

| | | | |
|---|---|---|---|
| ^{a}Direct analysis and (or) from literature. | | | |

**Pharmacokinetic Studies. PK of the compound of Formula I.** For intravenous infusion, the Formula I compound was dissolved in PEG 400/5% dextrose (7/3) at a concentration of 5 mg/ml. The solution was stable at room temperature. The compound was infused via the cephalic vein in the dog's front leg over a period of 30 min at a rate of 2 ml/kg/hr. Healthy female beagle dogs weighing from 8-10 kg were used for these studies. Blood samples (∼ 5 ml) for pharmacokinetic analysis were collected from a jugular vein over EDTA anticoagulant at selected times over 24 hr. Plasma was separated and frozen at -20°C to await analysis. For oral administration, the compound of Formula I was dissolved in PEG 400/Tween 20 (9/1) at a concentration of 25 mg/ml and loaded into hard gelatin capsules immediately prior to dosing. The dosing volume was 0.32 ml/kg. The oral dose was followed by 10 ml of water. The compound of Formula I was quantitated in plasma at MDS Analytical Solutions, Inc. (Sunnyvale, CA) using a validated HPLC procedure. The method employed reverse-phase chromatography with UV detection at 310 nm. The limit of quantitation was validated at 25 ng/ml of the compound of Formula I in plasma. Pharmacokinetic analysis was performed using standard non-compartmental methods.

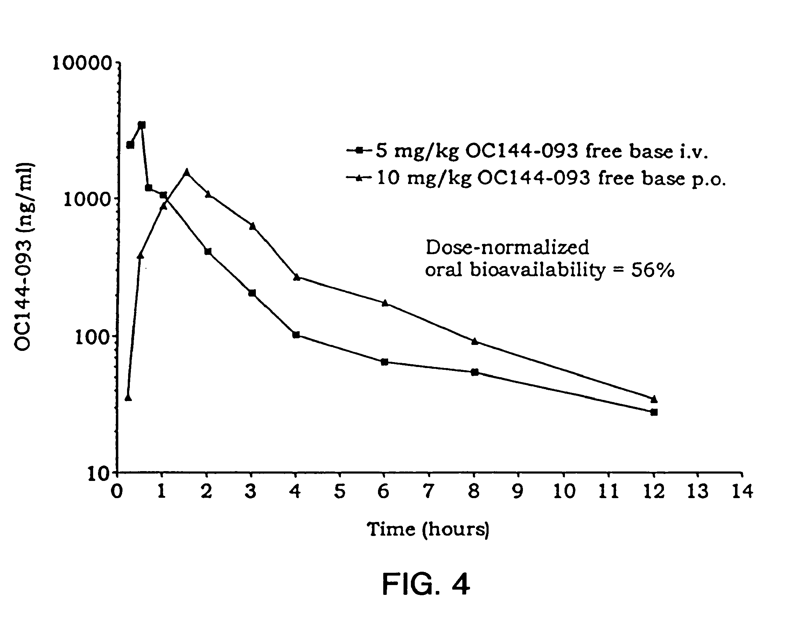

The compound of Formula I was orally bioavailable in rodents (mice and rats) and dogs (50-70%) and was well tolerated, with a terminal-half life of 4-7 hours (Figure 4). Mean total plasma clearance (CL) in the dog was 1.37 L/hr/kg and the mean volume of distribution at steady-state (Vss) was 3.24 L/kg in the dog. The maximum tolerated dose for oral compound in the dog was 60 mg/kg/day. Ataxia was the only effect observed at this dose, with complete recovery within 24 hours. There was no target organ toxicity observed during histopathological examination following i.v. administration at the MTD.

Blood levels >1 µM of the Formula I compound were observed in the dog for approximately 3 hours after an oral dose of 10 mg/kg (Figure 4), demonstrating that therapeutically relevant levels can be attained with a wide safety margin. In mouse studies, administration of three 20 mg/kg oral doses of the Formula I compound over a 24 hour period produced blood levels of compound of 2-4 µM, which were sustained for an additional 24 hours. These results demonstrate that therapeutically relevant doses of this compound can be administered via the oral route.

**Pharmacokinetic Studies. Oral Paclitaxel Bioavailability Enhancement.** A total of 21 male Sprague Dawley rats were used. Animals were approximately 8 weeks of age at arrival and weighed ca 327-359 g. Four males were randomly assigned to each of Groups 1 and 2. On the day of dosing and following an overnight fast, animals were weighed (ca 308-326 g) and administered their respective combination of doses. Animals of Group 1 received an oral dose of the compound of Formula I mesylate salt (10 mg of the free base/kg) by gavage at a dose volume of 1 mL/kg. Animals of Group 2 received the mesylate salt vehicle (deionized water) by gavage at a dose volume of 1 ml/kg. Sixty minute later, all animals in Groups 1 and 2 received a dose of Paclitaxel (40 mg/kg) by gavage at a dose volume of 2 ml/kg and a concomitant dose of either the Formula I compound or vehicle.

Following administration of paclitaxel, blood samples (ca 0.3 ml in EDTA were obtained from all rats by jugular venipuncture during anesthesia with isolurane (Abbott Laboratories) at each of the following time points: pre-dose (prior to administration of the Formula I compound or vehicles) and at 0.25, 0.5, 1, 2, 4, 6, and 8 hours following administration of the paclitaxel dose. Immediately following collection, all samples were placed on ice until further processing or storage. Blood samples were centrifuged (ca 3200g at about 4 °C for 10 minutes) and the resulting plasma samples were stored at -20 °C pending analysis for unchanged paclitaxel. Following analysis of paclitaxel, all remaining plasma were tested for a determination of the Formula I compound concentration.

Plasma samples were analyzed for unchanged paclitaxel using an LC/MS assay. Pharmacokinetic analysis was performed on plasma concentrations using the PhAST Software Program, Version 2.2-00 (Phoenix International Life Sciences, Inc.). The highest observable concentration was used as the peak concentration (Cₘₐₓ). Time to Cₘₐₓ was denoted as T_{max.} The area under the plasma concentration vs. time-curve from time zero to the last measurable concentration (AUC(0-t)) was calculated by the linear trapezoidal method. the area under the plasma concentration curve extrapolated to infinity (AUC(I)) was calculated as the sum of AUC(0-t) plus the ratio of the last plasma concentration to the elimination rate constant. The terminal elimination constant (K) was calculated from the last 3 non-zero points of the log-linear regression. Half-life (t^{1/2}) was determined by dividing 0.693 by K. The apparent plasma clearance (CL/F) was calculated for paclitaxel by dividing the nominal dose administered by AUC(I). Where applicable, numerical values were subjected to calculation of group means and %CV. Where values were below the limit of quantification, zero was used for pharmacokinetic analysis. Statistical analysis consisted of a Student-T-test with Two-sample unequal variance approximation and was performed on AUC90-t) and Cₘₐₓ values to compare the Formula I compound treated animals vs. the corresponding vehicle treated animals.

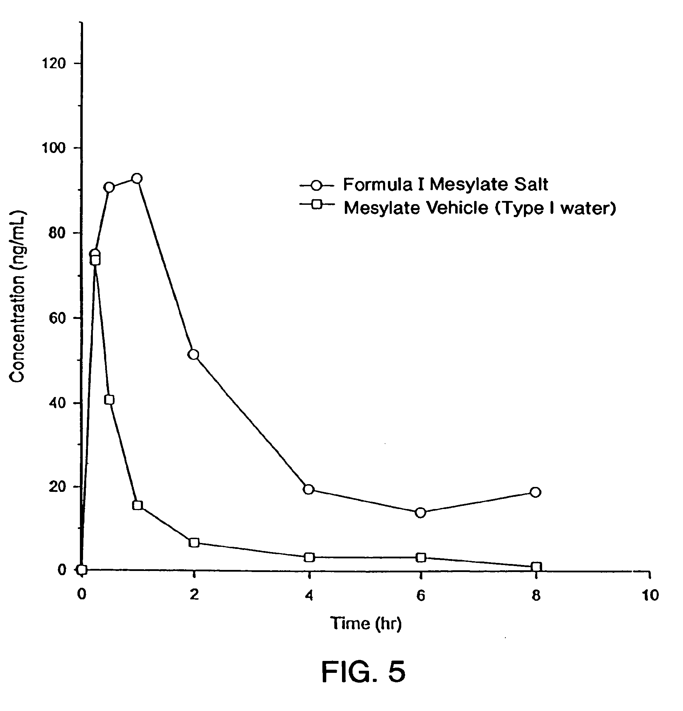

Linear graphical representations of the concentration of paclitaxel in plasma vs. time are presented in Figures 5 paclitaxel/mesylate salt.

Mean terminal phase half-life (t^{1/2}) values for paclitaxel were calculated to be 3.5 and 3.9 hr for Groups 1 and 2, respectively, suggesting a relatively moderate elimination of the parent drug following oral administration. The mean AUC(0-t) and AUC(I) values for paclitaxel in plasma was higher in both groups receiving the compound of Formula I 60 minutes prior to and concomitantly with the administration of paclitaxel. The mean AUC(0-t) values for Group 1 (mesylate salt) were 286 ng hr/ml. The values for the corresponding control group were 67 ng hr/ml (Group 2). The difference in AUC (0-t) between Group 1 and Group 2 was statistically different (P=0.003).

Mean Cₘₐₓ values were also higher for both Formula I treated groups when compared to their corresponding vehicles. These differences, however, were not statistically significant (P=0.22 for the mesylate salt form). These results suggest that the mesylate salt of Formula I significantly increased the oral bioavailability of paclitaxel. The AUC(0-t) value was over 4 times higher for the mesylate salt animals than for their vehicle treated counterpart.

**PK Interaction Studies.** Plasma paclitaxel levels were determined in SCID/RAG2 mice following pre-treatment with 3 p.o. gavage doses of 30 mg/kg Formula I compound (free base) or vehicle (100 µl PEG 400/Tween 20; 9/1 by volume) 25, 19 and 1 h prior to a single i.v. dose of 16 mg/kg paclitaxel in 200 µl saline/Cremophor/ethanol (8/1/1 by volume). At the indicated times after paclitaxel administration, 3 mice per time point were anesthetized with CO₂ and blood was removed by cardiac puncture into microtainer tubes containing EDTA. Plasma samples generated by centrifugation were extracted with acetonitrile and the analysis for paclitaxel content was carried out by HPLC using a Waters 600E multisolvent delivery system, 717 plus autosampler and 996 photodiode array detector. Baccatin III (0.8 nmoles/200 µl plasma) was used as the internal standard (IS) and added to plasma samples during extraction with acetonitrile. Standard curve samples as well as quality control samples were also prepared from spiked control plasma in order to verify the accuracy of the HPLC analysis. Paclitaxel and the IS were resolved on a Nova-Pak C₁₈ column (4 µm, 150 mm x 3.9 mm inside diameter; Waters, Milford, MA) with double distilled water (A), and acetonitrile (B), using the following gradient profile: t = 0 min, 10% B; t = 5 min, 10% B; t = 30 min, 65% B; t = 40 min, 65% B; t = 45 min, 10% B; t = 50 min, 10% B. The gradient was formed using a high pressure mixer and the flow rate was 1.0 ml min⁻¹. A Waters 996 Photo Diode Array Detector was used to scan at multiple wavelengths and chromatograms were processed for traces obtained at 230 nm.

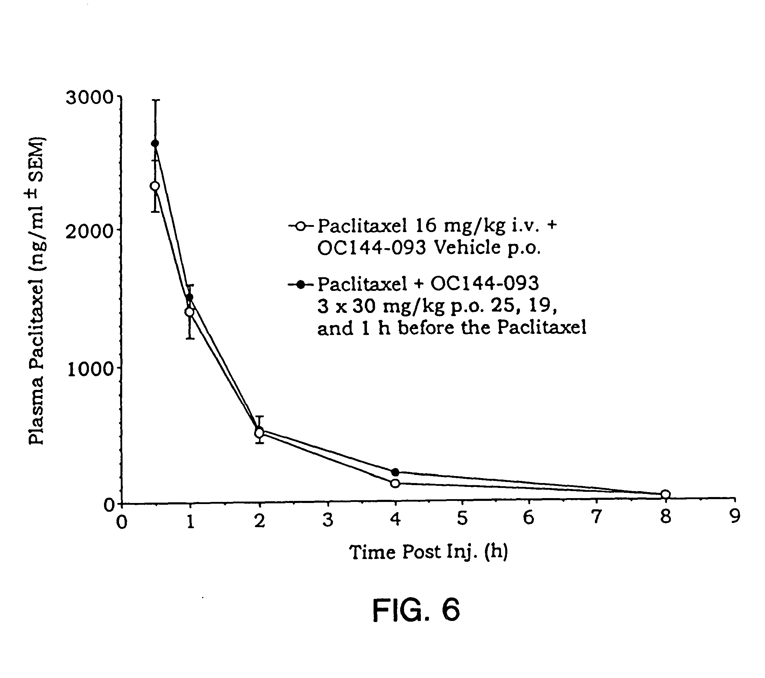

Two enzymes primarily responsible for metabolism of paclitaxel are P450 CYP3A4 and CYP2C8. Some P-gp inhibitors are also metabolized by P450 CYP 3A4, leading to inhibition of paclitaxel metabolism. This may contribute to PK interactions with paclitaxel. The Formula I compounds was not metabolized by P450 CYP3A4 or CYP2C8. The Kᵢ for the compound of Formula I inhibition of human CYP3A4-mediated paclitaxel metabolism was found to be 39.8 ± 5.1 µM. This is approximately 1000-fold higher than the EC₅₀s for reversal of MDR by the Formula I compound, suggesting that the compound might not produce a significant PK interaction with paclitaxel *in vivo.* Figure 6 demonstrates that pretreatment of mice with three oral doses of 30 mg/kg the Formula I compound had no effect on i.v. plasma paclitaxel levels.

**Cell Lines, Animals and Reagents.** CCRF-CEM and CEM/VLB 1000 human lymphoma, SKOV3 and SKVLB1000 human ovarian carcinoma cells from V. Ling (Vancouver, BC) were grown in Alpha MEM with 2.0 mM glutamine and 10% FBS (Gemini BioProducts, Calabasas, CA), plus 1.0 µg/ml vinblastine sulfate for maintenance of drug resistance. MCF-7 and MCF-7/ADR (NCI, DCT Tumor Repository) and MCF-7/VP human breast carcinoma cells (E. Schneider, Albany, NY) were grown in RPMI 1640 with 10% FBS. MES-SA and MES-SA/DX5 human uterine carcinoma cells (ATCC) were grown in McCoy's 5A with 10% FBS, plus 500 ng/ml doxorubicin for maintenance of drug resistance. MDA/LCC6 and *mdr1* transduced MDA/LCC6^{MDR1} human breast carcinoma cells from R. Clarke (Georgetown University, Washington, DC) were grown in IMEM with 5% FBS. HCT-15 human colon carcinoma cells (ATCC) were grown in RPMI 1640 with 10% FBS. P-gp expression or lack thereof in cell lines was confirmed by FACS analysis using the monoclonal antibody MRK16 (Kamiya Biomedicals, Berkeley, CA). Nontransformed HISM human smooth muscle and primary CCD-986SK human skin cells (ATCC) were grown in DME with 10% FBS and Iscove's modified Dulbecco's medium with 10% FBS respectively.

Six to eight week old female BDF1 and SCID/RAG2 mice were obtained from Charles River Laboratories of Canada and the Joint Animal Care Facility at the BC Cancer Research Centre, respectively. MDA/LCC6 and MDA/LCC6^{MDR1} cells (10⁷ in 0.5 ml) were similarly propagated every 2-3 weeks in SCID/RAG2 mice. Cells were used between the 3^{rd} and 20^{th} passage. Animal studies were carried out with protocols approved by the BCCA/University of British Columbia Institutional Animal Care Committee and were performed in accordance with the Canadian Council on Animal Care Guidelines. The *in vivo* HCT-15 study was conducted by Serquest, a division of Southern Research Institute (Birmingham, AL), with young, adult female athymic NCr-nu mice.

Vinblastine, doxorubicin, daunomycin, and verapamil were purchased from Fluka (Ronkonkoma, NY). Paclitaxel and Cyclosporin A were from Sigma (St. Louis, MO). Alamar Blue was from BioSource International (Camarillo, CA) and was used according to the manufacturer's instructions. The compound of Formula I-free base for *in vivo* studies was synthesized by IRIX Pharmaceuticals, Inc. (Florence, SC). The material was 98% pure as judged by HPLC.

While the invention has been described in detail with reference to certain preferred embodiments thereof, it will be understood that modifications and variations are within the spirit and scope of that which is described and claimed.

## Claims

1. Use of a compound according to formula I, derivatives or salts thereof for the preparation of a medicament for the treatment of a cell proliferative disorder, wherein the cell proliferative disorder is further treated by a chemotherapeutic agent.

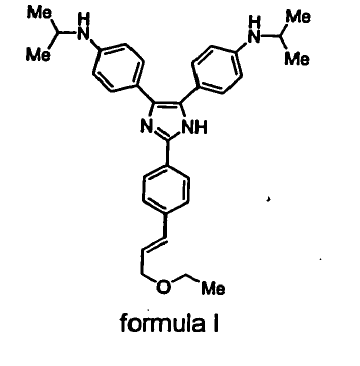

2. The use of a compound according to claim 1 for the preparation of a medicament to be administered simultaneously with the chemotherapeutic agent.

3. The use of a compound according to claim 1 for the preparation of a medicament to be administered after administration of the chemotherapeutic agent.

4. The use of a compound according to claim 1 for the preparation of a medicament to be administered prior to the administration of the chemotherapeutic agent.

5. The use of a compound according to claim 1 for the preparation of a medicament to be administered to a subject which is naive to the chemotherapeutic agent.

6. The use of a compound according to claim 1 for the preparation of a medicament to be administered to a subject which is not naive to the chemotherapeutic agent.

7. The use according to claim 1, wherein the cell proliferative disorder is a fibrotic disorder.

8. The use according to claim 1, wherein the cell proliferative disorder is a neoplasm or a cancer.

9. The use according to claim 8, wherein the cancer is a cancer of the breast, lung, prostate, kidney, skin, neural, ovary, uterus, liver, pancreas, epithelial, gastric, intestinal, exocrine, endocrine, lymphatic, hematopoietic system or a head and neck tissue.

10. The use according to claim 1, wherein the chemotherapeutic agent is selected from the group consisting of a taxane, a vinca alkaloid, an anthracycline, an epipodophyllotoxin, a dactinomycin, a mithromycin and a mitomycin.

11. The use according to claim 1, wherein the chemotherapeutic agent is administered at a standard dose.

12. The use according to claim 1, wherein the chemotherapeutic agent is administered at a dose of about 25 to 100% above standard levels.

13. The use according to claim 1, wherein the cells of the neoplasm do not express P-gp.

14. Use of a compound according to formula I or derivatives or salts thereof for the preparation of a medicament to be administered to a patient for inhibiting the emergence of multi-drug resistant (MDR) neoplastic cells with respect to the treatment of a patient with a chemotherapeutic agent, wherein the neoplastic cells do not express P-gp, do not express P-gp in all cells, or do not express P-gp at levels sufficient to manifest complete multidrug resistance.

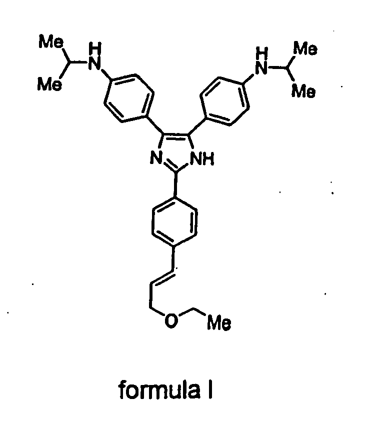

15. Use of a compound according to formula I or derivatives or salts thereof for the preparation of a medicament to be administered to a patient for the protection from toxicity elicited by a chemotherapeutic agent.
